(19) Europäisches Patentamt
European Patent Office
Office européen des brevets

(11) EP 3 366 712 A1

(12) # EUROPÄISCHE PATENTANMELDUNG

(43) Veröffentlichungstag:
**29.08.2018 Patentblatt 2018/35**

(21) Anmeldenummer: **17158249.7**

(22) Anmeldetag: **27.02.2017**

(51) Int Cl.:
*C08G 2/14* (2006.01) *C08G 61/12* (2006.01)
*B32B 21/04* (2006.01) *B27N 3/02* (2006.01)
*B27N 3/00* (2006.01) *C08L 97/02* (2006.01)
*C09J 161/06* (2006.01) *C09J 161/12* (2006.01)
*C09J 161/24* (2006.01) *C09J 161/28* (2006.01)
*C09J 161/34* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **AVALON Industries AG**
**6304 Zug (CH)**

(72) Erfinder:
- **BADOUX, François**
  **6314 Unterägeri (CH)**
- **KOEHLER, Stefan**
  **78467 Konstanz (DE)**
- **MORTATO, Mariangela**
  **4051 Basel Stadt (CH)**
- **KRAWIELITZKI, Stefan**
  **6343 Holzhäusern (CH)**

(74) Vertreter: **Geitz Truckenmüller Lucht Christ Patentanwälte PartGmbB**
**Obere Wässere 3-7**
**72764 Reutlingen (DE)**

(54) **NEUE HMF-OLIGOMERE**

(57) Die Erfindung betrifft neue kohlenstoffverknüpfte 5-Hydroxymethylfurfural(HMF)-Oligomere, enthaltend zumindest eine erste HMF-Einheit und eine zweite HMF-Einheit, die dadurch gekennzeichnet sind, dass erste und zweite HMF-Einheit über eine Kohlenstoff-Kohlenstoff-Bindung unter Einbeziehung eines aromatisch gebundenen Kohlenstoffatoms an Position 3 oder 4 des Furanrings der ersten HMF-Einheit verknüpft sind. Die Erfindung betrifft ferner die Verwendung der neuen HMF-Oligomere als reaktive Carbonylverbindungen in der Herstellung von thermisch härtbaren Harzen auf der Basis von phenolischen Verbindungen und/oder Aminoplastbildnern sowie in der Herstellung von Holzverbundwerkstoffen.

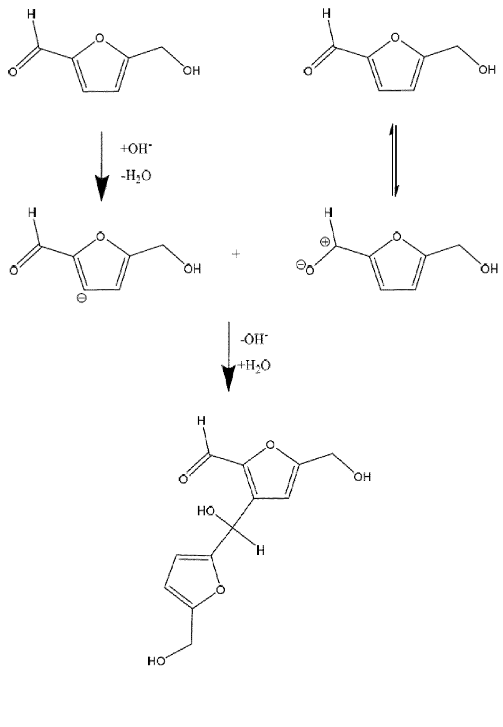


Fig. 2

EP 3 366 712 A1

## Beschreibung

Die vorliegende Erfindung betrifft neue HMF-Oligomere und Verfahren zur Herstellung derselben. Die vorliegende Erfindung betrifft im Besonderen neue kohlenstoffverknüpfte HMF-Oligomere. Diese sind nützlich als reaktive Carbonylverbindungen in der Herstellung von thermisch härtbaren Harzen auf der Basis von phenolischen Verbindungen und/oder Aminoplastbildnern sowie in der Herstellung von Holzverbundwerkstoffen.

5-Hydroxymethylfurfural (HMF)

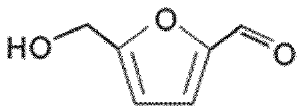

ist eine wichtige Plattformchemikalie und hat als Ausgangsverbindung für zahlreiche Synthesen erhebliche Bedeutung erlangt. Es ist zudem bekannt, bei der Herstellung von thermisch härtbaren Harzen auf der Basis von phenolischen Verbindungen und/oder Aminoplastbildnern gesundheitsschädliche reaktive Carbonylverbindungen wie Formaldhyd zumindest teilweise durch HMF zu ersetzen. In der Fachzeitschrift European Journal of Wood Products wird ein HMF-modifiziertes Harnstoff-Formaldehyd-Harz beschrieben, bei dessen Herstellung bis zu etwa 30 Gew.% des Formaldehyds durch gereinigtes, kristallines HMF ersetzt wurden (N. Esmaeili et al., DOI 10.1007/s0017-016-1072-8).

Das Auftreten von linearen und verzweigten Oligomeren in Lösungen von HMF ist bekannt, beispielweise aus der DE 10 2014 112 240 A1. Die HMF-Oligomere entstehen unter anderem bei der Herstellung von HMF aus Kohlenhydraten und kohlenhydrathaltiger Biomasse unter hydrothermalen Bedingungen und können durch NMR-, IR- und Massen-Spektroskopie nachgewiesen werden. Ihre Bildung kann zudem mittels HPLC- Analysen verfolgt werden.

Bislang bekannte oligomere Verbindungen aus HMF resultieren aus der Verknüpfung von Aldehyd- und/oder Hydroxylgruppen einzelner HMF-Monomere oder einzelner Monomere mit aus HMF-Monomeren bestehenden HMF-Oligomeren. Die HMF-Monomere stellen schließlich die Einheiten der gebildeten HMF-Oligomere dar. Die HMF-Oligomere sind linear, mehr oder weniger stark verzweigt und weisen Ether-, Hemiacetal- und/oder Acetal-Bindungen auf. HMF-Oligomere werden sowohl unter sauren als auch unter basischen Bedingungen gebildet.

Lineare HMF-Oligomere enthalten üblicherweise Strukturelemente, die durch Ether-Bindungen verknüpfte Einheiten des Typs

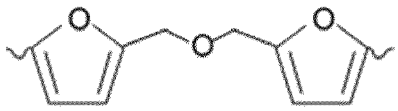

und/oder durch Bildung von Halbacetalen verknüpfte Einheiten des Typs

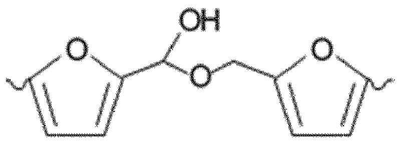

umfassen. Verzweigte HMF-Oligomere können zudem Strukturelemente mit unter Acetalbildung verknüpften Einheiten des Typs

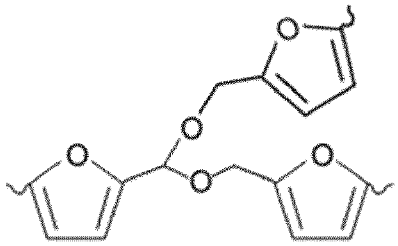

enthalten. Die geschwungenen Linien deuten dabei an, dass es sich bei den dargestellten Strukturelementen um einen Ausschnitt aus einem HMF-Oligomer handelt. Ein HMF-Oligomer weist in der Regel mehrere gleiche oder unterschiedliche Strukturelemente der angegebenen Typen auf. Endständige HMF-Einheiten werden von Aldehyd- oder Hydroxymethylgruppen begrenzt.

Die Erfinder haben herausgefunden, dass sich neben HMF-Oligomeren mit den Ether-, Hemiacetal- und/oder Acetal-Bindungen sowohl unter sauren als auch unter basischen Bedingungen kohlenstoffverknüpfte HMF-Oligomere bilden, bei denen Einheiten über eine Kohlenstoff-Kohlenstoff-Bindung verknüpft sind. Diese Bindungen können beispielsweise bei einem elektrophilen Angriff einer Aldehydgruppe eines ersten HMF-Monomers oder einer HMF-Einheit eines HMF-Oligomers auf das Kohlenstoffatom in Position 3 oder 4 eines Furanrings eines zweiten HMF-Monomers oder einer HMF-Einheit eines HMF-Oligomers entstehen.

Gegenstand der vorliegenden Erfindung sind daher neue kohlenstoffverknüpfte HMF-Oligomere, enthaltend zumindest eine erste HMF-Einheit und eine zweite HMF-Einheit, die sich dadurch auszeichnen, dass erste und zweite HMF-Einheit über eine Kohlenstoff-Kohlenstoff-Bindung unter Einbeziehung eines aromatisch gebundenen Kohlenstoffatoms an Position 3 oder 4 des Furanrings der ersten HMF-Einheit verknüpft sind.

Als "HMF-Oligomere" im Sinne der vorliegenden Erfindung werden im Unterschied zu den HMF-Monomeren Verbindungen aus mindestens zwei verknüpften HMF-Einheiten/Monomeren bezeichnet. Der Übergang zwischen HMF-Oligomeren und HMF-Polymeren ist dabei fließend. Im Sinne der vorliegenden Erfindung versteht man unter "HMF-Oligomeren" Verbindungen mit einem Molekulargewicht von bis zu 3000 g/mol.

HMF-Oligomere werden im Sinne der vorliegenden Erfindung als "kohlenstoffverknüpfte HMF-Oligomere" bezeichnet, sofern zumindest zwei HMF-Einheiten über eine Kohlenstoff-Kohlenstoff-Bindung unter Einbeziehung eines aromatisch gebundenen Kohlenstoffatoms an Position 3 oder 4 des Furanrings einer der beiden HMF-Einheiten verknüpft sind.

Nach einer vorteilhaften Ausgestaltung der Erfindung ist das aromatisch gebundene Kohlenstoffatom der ersten Einheit mit dem Aldehydkohlenstoffatom der zweiten HMF-Einheit verknüpft.

Die für die unter Beteiligung von aromatisch gebundenem Kohlenstoffatom und Aldehydkohlenstoffatom stattfindende Bildung einer Kohlenstoff-Kohlenstoffbindung im Sauren und im Basischen vorgeschlagenen Mechanismen können den Figuren 1 und 2 entnommen werden. Aus diesen wird unter anderem ersichtlich, dass HMF-Oligomere, die über eine Verknüpfung durch eine Kohlenstoff-Kohlenstoff-Bindung verfügen, gleichzeitig auch über mehr freie funktionelle Aldehyd- und/oder Hydroxyl-Gruppen verfügen als HMF-Oligomere, bei denen die Verbindung lediglich über Aldehyd- und/ Hydroxylgruppen des HMFs zustande kommt. Ursächlich ist dabei bevorzugt auch ein höherer Vernetzungsgrad, der auf der zusätzlichen Kohlenstoff-Kohlenstoff-Bindung beruht, wodurch eine höhere Dichte an freien Aldehyd- und Hydroxygruppen in einem HMF-Oligomer erreicht werden kann.

Nach einer weiteren vorteilhaften Ausgestaltung der Erfindung weist das kohlenstoffverknüpfte HMF-Oligomer 2 bis 20 HMF-Einheiten auf, bevorzugt 2 bis 10 HMF-Einheiten, besonders bevorzugt 2 bis 4 HMF-Einheiten. HMF-Oligomere mit 2 bis 10 Einheiten sind unter moderaten Bedingungen, das heißt bei Raumtemperatur und unter Normaldruck, gut wasserlöslich.

Nach einer weiteren vorteilhaften Ausgestaltung der Erfindung enthält das kohlenstoffverknüpfte HMF-Oligomer neben der unter Einbeziehung eines aromatisch gebundenen Kohlenstoffs verknüpften ersten und zweiten HMF-Einheit mindestens eine weitere Einheit, die unter Ausbildung einer Ether-, Hemiacetal- oder Acetal-Bindung mit einer HMF-Einheit verknüpft ist.

Ein weiterer Gegenstand der vorliegenden Erfindung ist ein Verfahren zur Herstellung der kohlenstoffverknüpften HMF-Oligomere, wobei das Verfahren beinhaltet, dass man eine wässrige Suspension von cellulosehaltiger Biomasse und/oder eine wässrige Kohlenhydrat-Lösung von mindestens einer Hexose und/oder eine wässrige 5-Hydroxymethylfurfural-Lösung unter hydrothermalen Bedingungen behandelt.

Die Behandlung von Biomasse wie pflanzlichen Rohstoffen, von Kohlenhydraten oder von aus Kohlenhydraten abgeleiteten Verbindungen unter hydrothermalen Bedingungen zur Gewinnung von 5-HMF(-Monomeren) ist bekannt und sieht vor, das Ausgangsmaterial in wässrigem Medium Druck und erhöhter Temperatur auszusetzen. Die Erfinder haben herausgefunden, dass bei der Behandlung einer wässrigen Suspension von cellulosehaltiger Biomasse und/oder einer wässrigen Kohlenhydrat-Lösung von mindestens einer Hexose und/oder einer wässrigen 5-Hydroxymethylfurfural-Lösung unter hydrothermalen Bedingungen kohlenstoffverknüpfte HMF-Oligomere gebildet werden.

Cellulosehaltige Biomasse, die häufig als Abfallprodukt der landwirtschaftlichen Erzeuger anfällt, ist aufgrund Ihres geringen Kostenfaktors besonders bevorzugt. Bevorzugte Hexosen sind Fructose oder Glucose, insbesondere kann es sich um Fructose oder Gemische aus Fructose und Glucose handeln.

Bevorzugte hydrothermale Bedingungen sind Sattdampfdruck und Temperaturen von 150 bis 250°C. Dies hat den Vorteil, dass die Bildung von HMF-Oligomeren in Abhängigkeit des Ausgangsmaterials innerhalb von Minuten bis zu wenigen Stunden abgeschlossen ist.

Ein weiterer Gegenstand der vorliegenden Erfindung ist ein Verfahren zur Herstellung der kohlenstoffverknüpften HMF-Oligomere, wobei das Verfahren enthält, eine mehr oder weniger reine Lösung von HMF-Monomeren und/oder HMF-Oligomeren Bedingungen auszusetzen, die zur Bildung von HMF-Oligomeren führen. Bevorzugt umfassen die Bedingungen, denen die HMF-Lösung ausgesetzt wird, eine Alkalisierung oder Ansäuerung und/oder ein Erhitzen der

Lösung.

Die Erfinder haben festgestellt, dass wässrige HMF-Lösungen, die beispielsweise aus kristallinem HMF mit Wasser hergestellt wurden, unter Bildung der HMF-Oligomere altern. Die Menge und das Molekulargewicht der HMF-Oligomere kann hierbei über dem Fachmann geläufige Analysemittel wie HPLC und NMR-Spektroskopie bestimmt werden.

Die Bildung von HMF-Oligomeren unter moderaten Bedingungen, das bedeutet, unter Normaldruck und Raumtemperatur, kann im Bereich von Stunden, Tagen oder Wochen liegen. Durch Ansäuerung, Alkalisierung und Erhitzen kann der Alterungsprozess beschleunigt werden.

Bevorzugt wird das Verfahren zur Herstellung so lange durchgeführt, bis die gewünschte Menge an kohlenstoffverknüpftem HMF-Oligomer erreicht ist oder die Reaktion abgeschlossen ist.

Es ist dabei für den Fachmann selbstverständlich, dass die Herstellung des kohlenstoffverknüpften HMF-Oligomers durch hydrothermale Behandlung mit der Herstellung des kohlenstoffverknüpften HMF-Oligomers durch Alterung kombiniert werden kann. So kann beispielsweise eine aus einer hydrothermalen Behandlung gewonnene wässrige Lösung anschließend bei Normaldruck gealtert werden.

Bevorzugt liegt das mindestens eine kohlenstoffverknüpfte HMF am Ende der Herstellung in wässriger Lösung vor.

Weiter bevorzugt ist es, den Gehalt, die Größe und/oder die Konzentration des kohlenstoffverknüpften HMF-Oligomers oder der kohlenstoffverknüpften HMF-Oligomere zu beeinflussen. Besonders bevorzugt wird der Gehalt des kohlenstoffverknüpften HMF-Oligomers oder der kohlenstoffverknüpften HMF-Oligomere beeinflusst, indem eine Lösung, enthaltend kohlenstoffverknüpfte HMF-Oligomere, einer Filtration an mindestens einem Filtriermittel unterzogen wird. Die Behandlung einer wässrigen HMF-Lösung nach einer hydrothermalen Karbonisierung durch Filtration wird beispielsweise in der DE 10 2014 112 240 A1 beschrieben.

Die kohlenstoffverknüpften HMF-Oligomere sind sehr gut geeignet als Komponenten in thermisch härtbaren Harzen, die vorzugsweise durch die Polykondensation von phenolischen Verbindungen und/oder Aminoplastbildnern mit reaktiven Carbonylverbindungen, insbesondere Aldehyden, gewonnen werden. Beispielhaft seien Aminoharze mit den Aminoplastbildnern Harnstoff, Melamin und Dicyandiamid, Phenol-Harze oder Amino-Phenol-Harze genannt. Durch anschließende Aushärtung der Harze erhält man einen duroplastischen Werkstoff.

Die kohlenstoffverknüpften HMF-Oligomere sind sehr reaktiv und verfügen über zusätzliche Vernetzungsmöglichkeiten. Sie sind sehr wirksam als reaktive Carbonylverbindung in thermisch härtbaren Harzen. Die erhaltenen Harze zeichnen sich durch besonders gute Verarbeitungseigenschaften wie eine sehr hohe Reaktivität aus. Ein großer Vorteil der Verwendung von kohlenstoffverknüpften HMF-Oligomeren zur Herstellung thermisch härtbarer Harzen ist, dass auf gesundheitsschädliche reaktive Carbonylverbindungen wie Formaldehyd vollkommen verzichtet werden kann.

Die Herstellung der thermisch härtbaren Harze durch Polykondensation erfolgt in an sich bekannter Weise. Geeignete Lösungsmittel sowie geeignete Reaktionsbedingungen wie beispielsweise Reaktionstemperatur und pH-Wert sind dem Fachmann grundsätzlich bekannt. Bevorzugt wird die Umsetzung in einem wässrigen Lösungsmittel durchgeführt.

Die kohlenstoffverknüpften HMF-Oligomere können in Gemischen mit weiteren reaktiven Carbonylverbindungen eingesetzt werden. Bevorzugt wird mindestens ein HMF-Oligomer in einem Gemisch mit mindestens einem HMF-Monomer und/oder mit mindestens einem weiteren HMF-Oligomer, enthaltend Ether-, Hemiacetal- und/oder Acetal-Bindungen, eingesetzt. Bereits geringe Mengen an kohlenstoffverknüpftem HMF-Oligomer reichen aus, um sehr reaktive Carbonylverbindungen bereitzustellen.

Die kohlenstoffverknüpften HMF-Oligomere eignen sich insbesondere zur Herstellung von Verbundwerkstoffen aus lignocellulosehaltigem Material wie Holzspänen, Holzfasern oder Holzchips. Die Herstellung der Holzverbundwerkstoffe erfolgt nach den auf diesem Fachgebiet allgemein bekannten Methoden. Die Holzverbundwerkstoffe können erhalten werden, indem man das lignocellulosehaltige Material mit thermisch härtbaren Harzen, enthaltend kohlenstoffverknüpfte HMF-Oligomere, in Kontakt bringt und die Harze anschließend aushärtet, was mit einer Vernetzung einhergeht. Die Aushärtung wird bevorzugt vorgenommen, indem man das mit dem lignocellulosehaltigen Material versehene Harz verpresst.

Die nachfolgenden Beispiele dienen lediglich der Erläuterung der Erfindung und sollen diese in keiner Weise beschränken.

Beispiel 1:

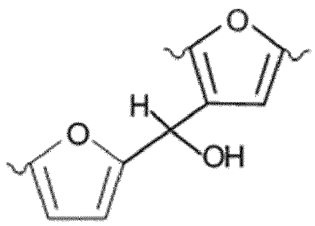

Beispiel 1 zeigt einen Ausschnitt aus einem kohlenstoffverknüpften HMF-Oligomer, enthaltend ein Strukturelement, das eine Kohlenstoff-Kohlenstoff-Bindung unter Einbeziehung eines aromatisch gebundenen Kohlenstoffatoms des Furanrings einer ersten HMF-Einheit und des Aldehydgruppenkohlenstoffatoms einer zweiten HMF-Einheit umfasst.

Der Übersichtlichkeit halber ist ein Ausschnitt aus dem kohlenstoffverknüpften HMF-Oligomer gezeigt, wie durch die geschwungenen Linien angedeutet. In Beispiel 1 nicht dargestellte endständige HMF-Einheiten werden bevorzugt von Aldehyd- oder Hydroxymethylgruppen begrenzt.

Beispiel 2:

Herstellung von Spanplatten

a) Bereitstellung einer HMF-Lösung mit HMF-Oligomeren:

Eine 16%tige wässrige Lösung aus kristallinem HMF wurde gleichzeitig konzentriert und gealtert, indem sie in einem Rotationsverdampfer bei 45°C und 30 mbar eingeengt wurde, bis die Konzentration an HMF 50 Gew.%, bezogen auf die Lösung betrug.

b) Herstellung und Vergleich der Eigenschaften von Harnstoff-HMF-Harzen:

Es wurden zwei Harze hergestellt, die sich in ihrem molaren Verhältnis von Harnstoff zu HMF unterschieden. Ein erstes Harz wurde mit einem Verhältnis von 1:0,5 Harnstoff zu HMF, im Folgenden mit UH(1:0,5) bezeichnet, hergestellt. Ein zweites Harz wurde mit einem Verhältnis von 1:0,25 Harnstoff zu HMF, im Folgenden mit UH(1:0,25) bezeichnet, hergestellt. Der Feststoffgehalt der Harze lag bei etwa 58%. Für beide Harze wurden 400 ml der 50%itigen HMF-Lösung aus a) verwendet. Bei beiden Harzen wurde der Harnstoff mit HMF bei einem pH-Wert von 2 zunächst für 2,5 Stunden und einer Temperatur von 90°C und anschließend mehrere Stunden bei einer Temperatur von 20°C umgesetzt. Die Änderung der Viskosität der Harze wurde dabei beobachtet.

Tabelle 1: Viskositätszunahme in Abhängigkeit der Zeit

| Zeit [Stunden] | Viskosität [mPa.s] | |
|---|---|---|
| | UH(1:0,5) | UH(1:0,25) |
| 4 | 470 | - |
| 24 | 1275 | 58 |
| 48 | - | 60 |
| 120 | - | 65 |
| 144 | - | 65 |
| 168 | - | 65 |

c) Verpressen von Holzspänen zu Spanplatten:

Für das anschließende Verpressen von Holzspänen wurde das Harz UH(1:0,5) mit einer Viskosität von 1275 mPa.s und das Harz UH(1:0,25) mit einer Viskosität von 65 mPa.s verwendet. Die Harze wurden jeweils mit den Holzspänen und mit Hexamethylentetramin vermischt und dann bei 220°C gepresst zur Herstellung von 250 mm x 250 mm x 16 mm großen Platten. Die Beladung des trockenen Holzes betrug 10 Gew.% Harzfeststoff, bezogen auf die Holzmenge. Um den Einfluss verschiedener Presszeiten und verschiedener Mengen an Härter zu testen, wurden mehrere Platten unter Variation der Zeiten und der Mengen an Hexamethylentetramin hergestellt. Die mit den beiden Harzen UH(1:0,5)

und UH(1:0,25) für die Spanplatten erhaltenen Werte sind in der Tabelle 2 angegeben.

Zum Vergleich wurde ein drittes Harz, UH45(1:0,5), hergestellt, indem die Komponenten des Harzes UH(1:0,5) bei einer tieferen Temperatur von 45°C umgesetzt wurden. Das Harz UH45(1:0,5) wurde ebenfalls für das Verpressen von Holzspänen zu 250 mm x 250 mm x 16 mm Spanplatten verwendet. Die für diese Spanplatten erhaltenen Werte sind ebenfalls in der Tabelle 2 angegeben.

Der Vergleich der mit den Harzen hergestellten Platten ergab, dass grundsätzlich bei einer längeren Pressdauer bessere Werte für die Innere Festigkeit erhalten werden.

Mit einem Molverhältnis von Harnstoff zu HMF von 1:0,5 erreichen die Platten 3 und 4 die hohen Werte von 52 N/mm$^2$ und 55 N/mm$^2$. Diese Werte sind auf eine Pressdauer von 7,5 min in Verbindung mit einer hohen Herstellungstemperatur der Harze von 90°C zurückzuführen.

Die Platten 1 und 2 sowie 5 und 6 verdeutlichen den Einfluss der Temperatur bei der Herstellung der Harze.

Selbst Platten, welche mit geringeren Mengen an HMF hergestellt wurden, liefern ein befriedigendes Ergebnis, wenn die Pressdauer erhöht wird, wie die Platten 7 bis 10 zeigen.

Hinsichtlich dem Härter hat sich gezeigt, dass sich unterschiedliche Härtermengen weniger stark bis nicht bemerkbar machen, sofern die Platten mit einem gewissen Anteil an HMF hergestellt wurden, wie die Platten 3 bis 6 zeigen. Die Platten 7 und 10 mit niedrigeren Anteilen an HMF werden deutlich stärker von der Härtermenge beeinflusst. Die Werte verdeutlichen, dass infolge der positiven Eigenschaften der verwendeten HMF-Oligomere die benötigten Härtermengen drastisch reduziert werden können, wobei Produkte mit identischer bzw. vergleichbarer Inneren Bindungsstärke erhalten werden können.

Innere Bindungsstärke (IB) gemäß NF EN 319 (AFNOR 1993):

Die innere Bindungsstärke in [N/mm$^2$] wird durch die folgende Formel ausgedrückt:

$$IB = \frac{Fmax}{axb},$$

wobei *Fmax* die Bruchkraft, *a* die Breite und *b* die Länge der Platte ist.

Die NF EN 319 (AFNOR 1993) sieht für Span- und Faserplatten mit einer Stärke im Bereich von 13 mm bis 20 mm eine Innere Bindungsstärke von ≤0,35 N/mm$^2$ vor.

Die Platten zur Untersuchung der Inneren Bindungsstärke wurden durch Zerschneiden aus den unter c) hergestellten Platten gewonnen. Ihre Größe betrug 50 mm x 50 mm. Vor dem Zerschneiden wurden die Platten in einem Trockner bei 20°C und einer relativen Luftfeuchtigkeit von 65% stabilisiert.

Die Platten wurden mit Hilfe eines Heißschmelzklebstoffs auf einer Unterlage befestigt. Die Bestimmung der Inneren Bindungsstärke fand gemäß NF EN 319 (AFNOR 1993) maschinell senkrecht zur Ebene der Platten statt.

Tabelle 2: Parameter der Spanplattenherstellung und Eigenschaften der Spanplatten

| Platte | Harz | Synthesetemperatur [°C] | Viskosität [mPa.s] | Molverhältnis Harnstoff:HMF | Presstemperatur [°C] | Pressdauer [min] | Härter [%] | Dichte [kg/m³] | Innere Bindungsstärke (IB) [N/mm²] |
|---|---|---|---|---|---|---|---|---|---|
| 1 | UH45 (1: 0,5) | 45 | 382 | 1:0.5 | 220 | 5.5 | 5 | 733 | **0.27** |
| 2 | UH45 (1: 0,5) | 45 | 382 | 1:0.5 | 220 | 5.5 | 2.5 | 729 | **0.21** |
| 3 | UH(1: 0,5) | 90 | 1275 | 1:0.5 | 220 | 7.5 | 5 | 717 | **0.55** |
| 4 | UH(1: 0,5) | 90 | 1275 | 1:0.5 | 220 | 7.5 | 2.5 | 718 | **0.52** |
| 5 | UH(1: 0,5) | 90 | 1275 | 1:0.5 | 220 | 5.5 | 5 | 715 | **0.43** |
| 6 | UH(1: 0,5) | 90 | 1275 | 1:0.5 | 220 | 5.5 | 2.5 | 718 | **0.43** |
| 7 | UH(1: 0,25) | 90 | 65 | 1:0.25 | 220 | 7.5 | 5 | 714 | **0.44** |
| 8 | UH(1: 0,25) | 90 | 65 | 1:0.25 | 220 | 6.5 | 5 | 715 | **0.39** |
| 9 | UH(1: 0,25) | 90 | 65 | 1:0.25 | 220 | 5.5 | 5 | 712 | **0.31** |
| 10 | UH(1: 0,25) | 90 | 65 | 1:0.25 | 220 | 7.5 | 2.5 | 713 | **0.36** |

Weitere Vorteile und vorteilhafte Ausgestaltungen sind den Ansprüchen und der nachfolgenden Zeichnung zu entnehmen.

Es zeigen

Figur 1 einen vorgeschlagenen Mechanismus der Kohlenstoff-Kohlenstoff-Bindungsbildung unter sauren Bedingungen anhand der Dimerisierung zweier HMF-Moleküle, sowie

Figur 2 einen vorgeschlagenen Mechanismus der Kohlenstoff-Kohlenstoff-Bindungsbildung unter basischen Bedingungen anhand der Dimerisierung zweier HMF-Moleküle.

Sämtliche Merkmale der Erfindung können sowohl einzeln als auch in beliebiger Kombination miteinander erfindungswesentlich sein.

## Patentansprüche

1. Neue kohlenstoffverknüpfte HMF-Oligomere, enthaltend zumindest eine erste HMF-Einheit und eine zweite HMF-Einheit, **dadurch gekennzeichnet, dass** erste und zweite HMF-Einheit über eine Kohlenstoff-Kohlenstoff-Bindung unter Einbeziehung eines aromatisch gebundenen Kohlenstoffatoms an Position 3 oder 4 des Furanrings der ersten HMF-Einheit verknüpft sind.

2. HMF-Oligomere nach Anspruch 1, **dadurch gekennzeichnet, dass** das aromatisch gebundene Kohlenstoffatom der ersten HMF-Einheit mit dem Aldehydkohlenstoffatom der zweiten HMF-Einheit verknüpft ist.

3. HMF-Oligomere nach Anspruch 1 oder 2, **dadurch gekennzeichnet, dass** das kohlenstoffverknüpfte HMF-Oligomer 2 bis 20 HMF-Einheiten, bevorzugt 2 bis 10 HMF-Einheiten, besonders bevorzugt 2 bis 4 HMF-Einheiten, aufweist.

4. HMF-Oligomere nach einem der vorhergehenden Ansprüche, **dadurch gekennzeichnet, dass** das kohlenstoffverknüpfte HMF-Oligomer neben der unter Einbeziehung eines aromatisch gebundenen Kohlenstoffs verknüpften ersten und zweiten HMF-Einheit mindestens eine weitere Einheit, die unter Ausbildung einer Ether-, Hemiacetal- oder Acetal-Bindung mit einer HMF-Einheit verknüpft ist, enthält.

5. Verfahren zur Herstellung der HMF-Oligomere, wobei das Verfahren beinhaltet, dass man eine wässrige Suspension von cellulosehaltiger Biomasse und/oder eine wässrige Kohlenhydrat-Lösung von mindestens einer Hexose und/oder eine wässrige 5-Hydroxymethylfurfural-Lösung unter hydrothermalen Bedingungen behandelt.

6. Verfahren zur Herstellung der HMF-Oligomere, wobei das Verfahren beinhaltet, dass man eine mehr oder weniger reine Lösung von HMF-Monomeren und/oder HMF-Oligomeren unter Normaldruck Bedingungen aussetzt, die zur Bildung von HMF-Oligomeren führen.

7. Verwendung der HMF-Oligomere nach einem der Ansprüche 1 bis 4 zur Herstellung thermisch härtbarer Harze.

8. Verwendung der HMF-Oligomere nach einem der Ansprüche 1 bis 4 zur Herstellung von Verbundwerkstoffen aus lignocellulosehaltigem Material.

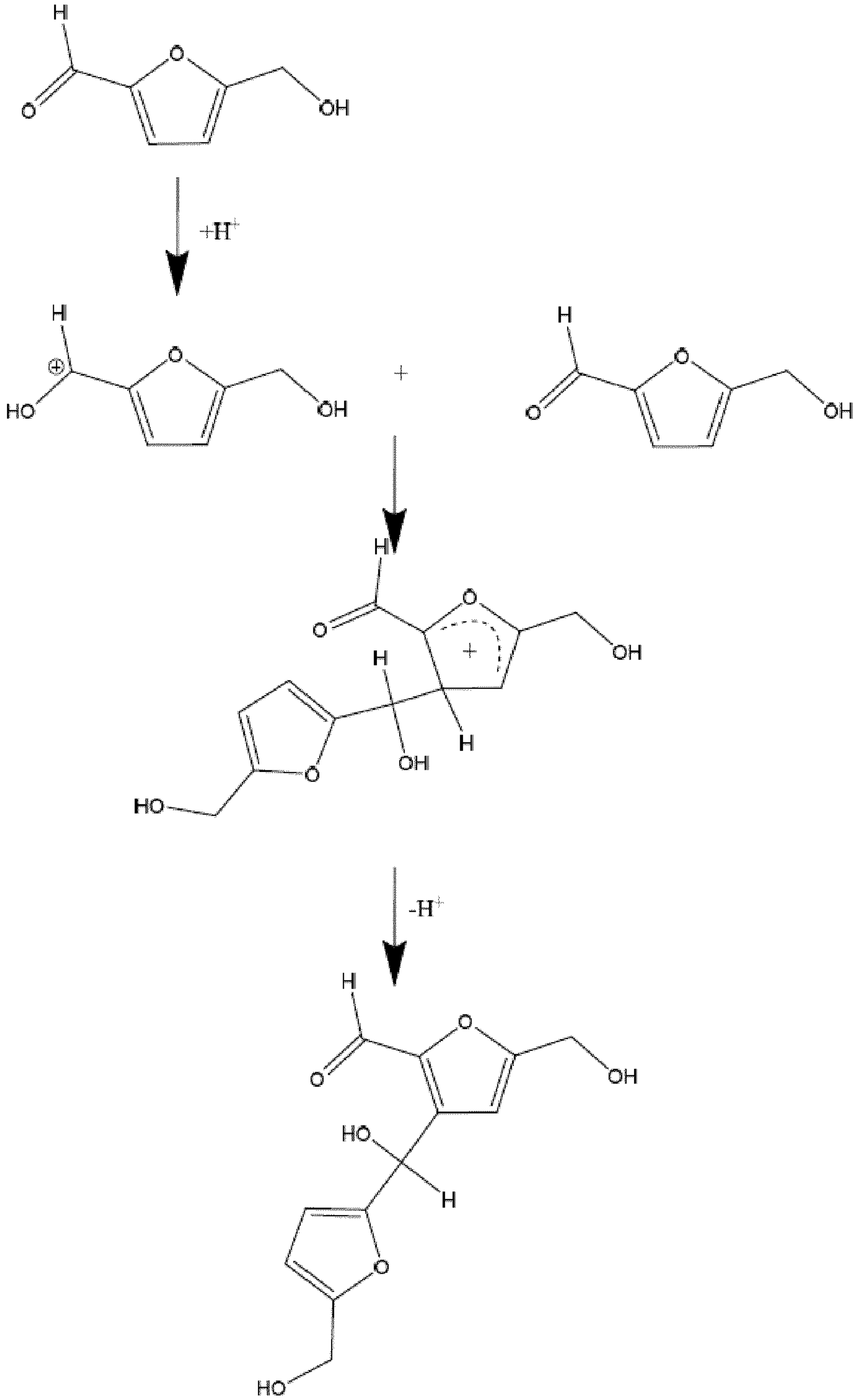


Fig. 1

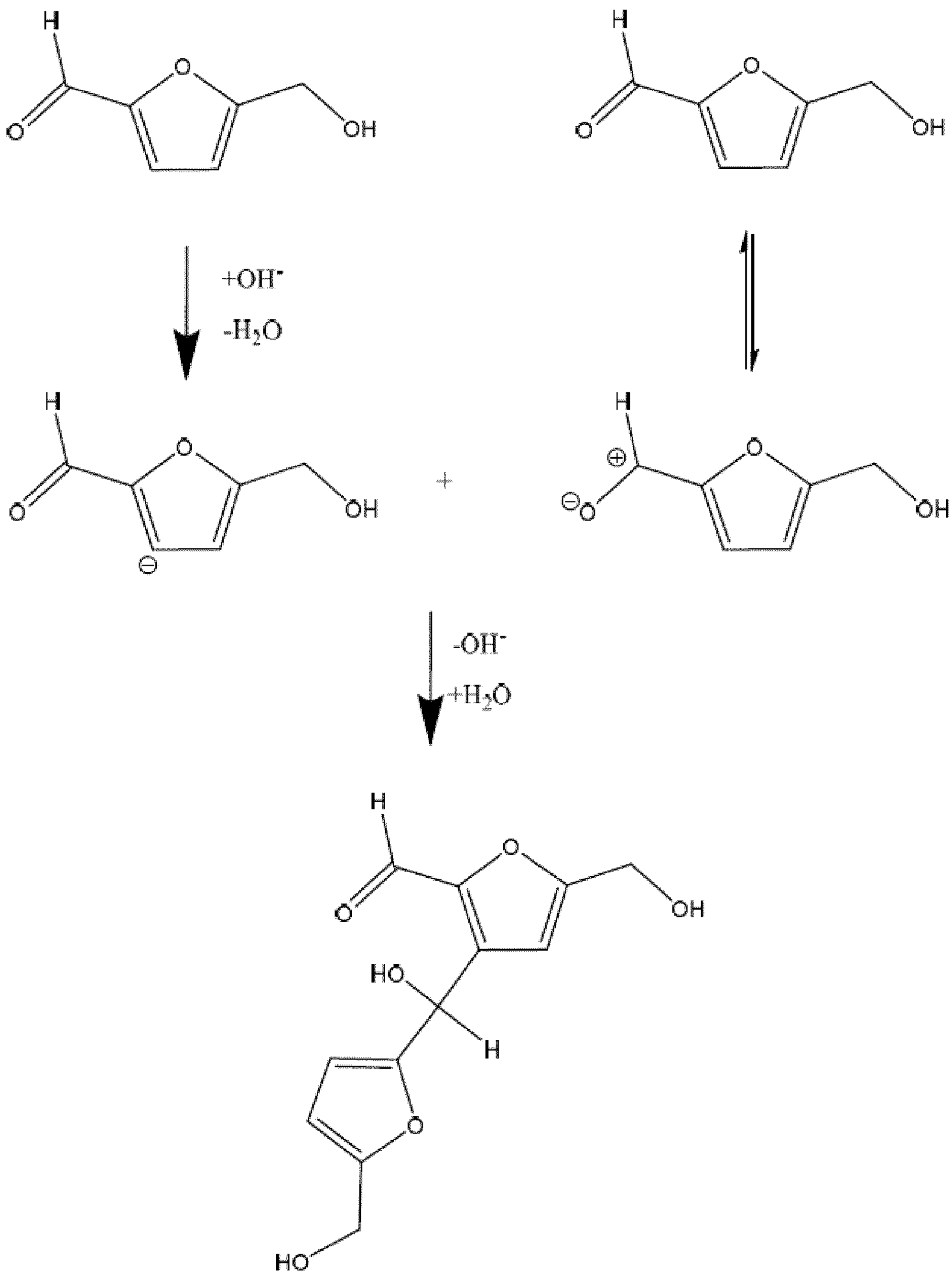


Fig. 2

Europäisches Patentamt
European Patent Office
Office européen des brevets

# EUROPÄISCHER RECHERCHENBERICHT

Nummer der Anmeldung
EP 17 15 8249

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X<br>Y | US 2 776 948 A (SNYDER FRANCIS H) 8. Januar 1957 (1957-01-08)<br>* Spalte 1, Zeile 49 - Zeile 59 *<br>* Spalte 2, Zeile 54 - Zeile 57 *<br>* Spalte 2, Zeile 54 - Zeile 66 *<br>* Spalte 4, Zeile 6 - Zeile 10 *<br>----- | 1-8<br>8 | INV.<br>C08G2/14<br>C08G61/12<br>B32B21/04<br>B27N3/02<br>B27N3/00<br>C08L97/02<br>C09J161/06<br>C09J161/12<br>C09J161/24<br>C09J161/28<br>C09J161/34 |
| X | DE 10 2014 112240 A1 (AVA CO2 SCHWEIZ AG [CH]) 3. März 2016 (2016-03-03)<br>* Seite 1, Absatz [0002] - Absatz [0003] *<br>----- | 1-5 | |
| Y | US 4 524 164 A (VISWANATHAN TITO [US] ET AL) 18. Juni 1985 (1985-06-18)<br>* Spalte 1, Zeile 10 - Zeile 13 *<br>----- | 8 | |
| | | | RECHERCHIERTE SACHGEBIETE (IPC) |
| | | | C08G<br>B32B<br>B27N<br>C08L<br>C09J |

Der vorliegende Recherchenbericht wurde für alle Patentansprüche erstellt

1

| Recherchenort | Abschlußdatum der Recherche | Prüfer |
|---|---|---|
| München | 24. Mai 2017 | Hofmeister, Ines |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 17 15 8249

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-05-2017

| Im Recherchenbericht angeführtes Patentdokument | Datum der Veröffentlichung | Mitglied(er) der Patentfamilie | Datum der Veröffentlichung |
|---|---|---|---|
| US 2776948 A | 08-01-1957 | KEINE | |
| DE 102014112240 A1 | 03-03-2016 | DE 102014112240 A1<br>EP 2990385 A1<br>TW 201607896 A<br>US 2016060141 A1 | 03-03-2016<br>02-03-2016<br>01-03-2016<br>03-03-2016 |
| US 4524164 A | 18-06-1985 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102014112240 A1